# EUROPÄISCHE PATENTANMELDUNG

(11) **EP 2 790 119 A1**
(43) Veröffentlichungstag der Anmeldung: **15.10.2014**
(21) Anmeldenummer: 13163301.8
(22) Anmeldetag: 11.04.2013
(51) Int. Cl.: G06F 19/00

(54) **System zur Durchführung von Übungen mit wählbaren Gewichten**

(71) Anmelder: MP Sports Coaching and Consulting GmbH, 80796 München (DE)
(72) Erfinder: Schmötzer, Immanuel, 80796 München (DE)
(74) Vertreter: Savic, Bojan

(57) **Zusammenfassung**

System zur Durchführung von Übungen mit wählbaren Gewichten umfassend: Einen scannbaren Code, der einem Fitnessgerät zugeordnet ist; einen Scanner, der durch einen Benutzer zum Scannen vom Code betätigt wird; ein Mittel, das durch das Betätigen des Scanners den Benutzer auf eine Website automatisch leitet; eine Datenbank, die mindestens eine Übung, die dem Code zugeordnet ist, enthält und; Anzeigen der Übung, die dem Code zugeordnet ist; das Mittel zur Eingabe des Gewichts und der Wiederholungszahlen; und ein Mittel zur Speicherung von Gewicht und von Wiederholungszahlen für die Übung, die dem Code zugeordnet ist.

## Beschreibung

Die vorliegende Erfindung betrifft das Gebiet der Übungen, bei denen Gewichte verwendet werden. Diese Übungen können Bestandteil der Sportarten wie z.B. Kraftsport, Body Building, Cross Fit, im Nachfolgenden als Kraftsportarten genannt, aber auch als Therapiemaßnahme zur Verbesserung der Muskelfunktion bei Patienten, Unfallopfer oder Leistungssportler.

Bei Kraftsportarten ist es wichtig, dass Übungen mit Gewichten in verschiedenen Belastungsintensitäten und mit entsprechenden Wiederholungszahlen korrekt ausgeführt und regelmäßig durchgeführt werden. Es ist dabei von Bedeutung, dass das Training variiert wird und die Übungen oft gewechselt werden, da sich der Körper an die immer gleichen Übungen sehr schnell gewöhnt. Die Übungen können entweder mit freien Gewichten, wie z.B. Gewichtsscheiben, Kurz- oder Langhanteln, Bällen, Säcken oder mittels der für die Kraftsportarten speziell entwickelten Geräten durchgeführt werden.

Die Übungen mit Gewichten können in Fitness- oder Therapiezentren durchgeführt werden. Wegen der Vielfalt der möglichen Übungen wird z.B. im Fitnessstudio üblicherweise ein Trainingsplan von einem Trainer erstellt, und dieser Plan wird dann in regelmäßigen Abständen vom Trainer überprüft und gegebenenfalls geändert. Der Trainingsplan beinhaltet eine begrenzte Zahl von verschiedenen Übungen, die je nach Wunsch und Bedarf zusammengestellt werden. Dabei wird die Zahl der Wiederholungen der jeweiligen Übungen entweder manuell oder elektronisch festgehalten. Um die Verletzung zu vermeiden und die Effizienz der Übungen zu erhöhen, ist es wichtig, dass die Übungen korrekt ausgeführt werden. Übungsvideos zum jeweiligen Fitnessgerät gibt es bereits auf der Webseite des Herstellers, als auch über Video-Streaming-Plattformen wie YouTube. Ebenso nutzen einige Hersteller QR-Codes auf ihren Geräten zur Verlinkung zu diesen Videos. Der QR-Code besteht aus einer quadratischen Matrix aus schwarzen und weißen Punkten, die z.B. eine Internet-URL-Adresse enthalten können. Durch das Scannen eines QR Code mittels eines Smartphones wird der Benutzer des Telefons direkt auf eine bestimmte Web-Site geführt.

Die Geräte des Herstellers werden auf Weblinks dargestellt und die korrekte Durchführung mit Videos veranschaulicht (http://www.precor.com/dede/commercial/products/strength/discovery-strength/selectorized/upperbody/biceps-curl-204).

Eine weitere verwandte Lösung sind vollautomatisierte Geräte mit Chip-Karten-Kennung. Der Benutzer nutzt seine Persönliche Chip-Karte (z.B. eine Mitgliedschaftskarte eines Fitnessstudios) zur Identifizierung. Das Fitnessgerät besitzt eine Schnittstelle und liest die gespeicherten Daten der Karte. Die Sitzeinstellungen und das Gewicht der letzten Einheit werden vollautomatisch eingestellt, der Benutzer beginnt zu trainieren.

Dabei werden alle Daten (Wiederholungen, Gewicht, Sitzeinstellungen, Datum) auf eine Webseite des Herstellers automatisch gespeichert. Über eine Webseite des Herstellers kann der Benutzer mit angelegtem Account die gespeicherten Daten einsehen.

Die Aufgabe der Erfindung besteht darin, ein Verfahren bereitzustellen, das ohne eine Chipkarte auskommt aber trotzdem für alle Geräte währen des Trainings eine Videounterstützung ermöglicht und gleichzeitig eine Tagebuchfunktion aufweist. Außerdem ist eine weitere Aufgabe der Erfindung ein Verfahren bereitzustellen, das eine Betreuung ohne persönlichen Kontakt zwischen dem Betreuer und dem Betreuten ermöglicht.

Die Lösung der Aufgabe ist durch die kennzeichnenden Merkmale des Anspruchs 1 angegeben.

Grundsätzlich umfasst das erfindungsgemäße System:
- einen scannbaren Code;
- einen Scanner;
- ein Mittel, der durch das Scannen des Codes den Benutzer auf eine Internet Website automatische leitet;
- eine Datenbank, die mindestens eine Übung, die dem Code zugeordnet ist, enthält, sodass diese Übung anzeigt wird gegebenenfalls mit dem Gewicht und Wiederholungszahlen, falls diese Übung vom Benutzer in der Vergangenheit ausgeführt wurde;
- ein Mittel zur Speicherung von Gewicht und von Wiederholungszahlen für die Übung, die dem Code zugeordnet ist.

Falls in der Datenbank mehrere Übungen einem Code zugeordnet sind, wird vom Benutzer eine dieser Übungen ausgewählt. Nach der Ausführung der Übung werden das Gewicht und die Wiederholungszahlen eingegeben und automatisch gespeichert.

Das erfindungsgemäße Verfahren ist eine webbasierte Anwendung zum Einsatz im Fitnessbereich. Inhaltlich eignet sich das Verfahren für einzelne Fitness- und Personal-Trainer, Athletiktrainern, Physiotherapeuten, Sporttherapeuten und Instruktoren - im Nachfolgenden Betreuer genannt - einerseits und zur Betreuung von Kunden, Sportlern und Patienten - im Nachfolgenden als Betreute genannt - andererseits. Die Betreuung kann z.B. in Fitnessstudios, Therapieeinrichtungen oder Vereinen stattfinden.

Durch das erfindungsgemäße Verfahren können die Verletzungen vermieden werden, da der Benutzer seine Daten einsehen kann und damit auch das in der Vergangenheit verwendete Gewicht. Darüber hinaus kann der Benutzer eine korrekte Ausführung einsehen.

In einer Ausführungsform ist für die Betreuer eine komplette Datenverwaltung mit persönlichen Daten und mit Anamnesebogen von Betreuten möglich. Darüber hinaus können die Betreuer von individuellen Trainingsplänen aus einer Datenbank von über 1200 Übungen, die Durchführung von verschiedenen Leistungstests im konditionellen Bereich, sowie die Erfassung der körperlichen Entwicklung anhand von anthropometrischen Messungen erstellen und übermitteln.

Vorzugsweise ist jede Übung ist mit verschiedenen Tipps zur Ausführung, mit mindestens einem vorzugsweise 2 Bildern und einem Video versehen. Mittels eines praktischen Filtersystems kann eine gewünschte Übung sehr schnell gefunden werden.

Alle diese Daten werden mittels Cloud-Service auf den Servern gespeichert und sind für die Betreuer und Betreuten immer und überall abrufbar und benutzbar.

Alle Accounts von Betreuten, die von einem Betreuer angelegt werden, werden z.B. über email-Adressen im System gespeichert und identifiziert. Der Betreuer hat somit die Möglichkeit neue Trainingspläne oder letzte Testergebnisse und Messungen direkt per email an die Betreuten zu senden.

Ebenfalls kann im Falle eines Einzeltrainings jede Trainingseinheit, mit jedem einzelnen Gewicht und jeder Wiederholung vom Trainer protokolliert werden. Alle relevanten Daten hat der Betreuer immer bei sich.

Registriert sich der Sportler mit derselben email-adresse im System, mit der er von seinem Trainer gespeichert worden ist, erhält er Zugang zu "gemeinsamen" Inhalten. Er kann damit sofort die für ihn persönlich erstellten Pläne einsehen. Auch hier sind pro Übung 2 Bilder und ein Video, und bis zu 8 Ausführungstipps und mögliche Fehler hinterlegt, und, wenn vorhanden, auch noch persönliche Anmerkungen seines Trainers. Er hat zudem Einsicht auf alle seine bereits absolvierten Trainingseinheiten bzw. auch die exakten Protokolle jeder einzelnen Übung mit Datum und erbrachter Leistung. Die selbst eingefügten Protokolle können sich farblich von den Protokolleinträgen des Trainers unterscheiden.

Möchte sich der Betreute nicht mit einem der ihm zugewiesenen Trainingspläne beschäftigen, stehen ihm auch hier die vorgefertigten Vorlagen mit unterschiedlichen Zielsetzungen zur Verfügung. Oder, um ganz frei zu wählen, hat er die komplette Übungsdatenbank zur Verfügung und kann mittels dem Filtersystem seine Lieblingsübungen suchen, auswählen und protokollieren.

Neben dem Abrufen von Übungen, Plänen, Vorlagen und Protokollen kann der Betreute zusätzlich noch auf seine Messdaten zurückgreifen. Bei der Messung von Körperumfängen und von Kalipermessungen kann er die fachspezifischen Messungen seines Trainers grafisch aufbereitet einsehen. Den weiteren Körperdaten (Größe, Gewicht, Körperfettanteil) kann er den Messungen seines Trainers seine eigene Messungen noch ergänzend hinzufügen und bearbeiten. Zusammenfassend stellt das erfindungsgemäße Verfahren ein interaktives und mobiles System für z.B. Fitness-Trainer und Sportler dar, auf dem von beiden Seiten aus miteinander gearbeitet wird und mit dem es möglich ist, die komplette Leistung des Sportlers, die Häufigkeit, die Intensität, also die gesamte Entwicklung bis ins letzte Detail zu protokollieren. Dieses Tracking-System hilft allen Beteiligten, ihr Ziel schneller zu erreichen.

Wie oben beschrieben, ist Kernstück einer jeden Trainingsplanung, von Trainerseite als auch in Eigenregie vom Sportler, die Übungsauswahl. Gleiches gilt für den Trainingsprozess und die Protokollierung.

Damit jeder Sportler über seine Trainings-App und auch jeder Trainer über seine Software schneller die gewünschte Übung finden kann, wird ein QR-Code Scanner, der z.B. in einem Smarphone integriert ist, verwendet.

Jede Übung bzw. jedes Gerät kann über einen spezifischen Link im Software-Code identifiziert werden. Jede Übung erhält daher einen eigenen QR-Code, der als Aufkleber an das jeweilige Fitnessgerät angebracht werden kann. Als Fitnessgeräte gelten hierbei sowohl feste Maschinen und Seilzüge mit ihren Varianten als auch Übungszusätze wie Kurz- oder Langhanteln, Kettlebells, Gymnastikbälle, Sling Trainer, Foam Roller, etc.

Der Kunde/Trainer scannt direkt an einem dieser Geräte diesen Code und erhält sofort alle Übungen, die an oder mit diesem Gerät durchführbar sind. Er hat damit sofortigen Einblick auf die Bilder, das Video, die Durchführungstipps und ganz wichtig: auf seine letzten Protokolle an bzw. mit diesem Gerät. Und zwar sind diese mit dem entsprechenden Datum, dem verwendeten Gewicht und den geleisteten Wiederholungen oder der erreichten Dauer versehen. Die zuletzt gezeigte Leistung ist voreingestellt und muss im Bedarfsfall nur geringfügig verändert werden. Mit einem Knopfdruck kann der neue Übungssatz gespeichert werden.

Fitnessstudios erhalten die QR-Code-Aufkleber inklusive der Software.

Ein Trainierender muss nicht registriert sein, um diesen Service zu nutzen. Jeder übliche und etablierte QR-Scanner für Smartphones kann die Aufkleber dekodieren und leitet den User direkt auf die mobile Trainingswebseite zu den hinterlegten Übungsvarianten mit Bildern und Videos.

Falls der Trainierende registrierter User ist, erhält er zuzüglich allgemeine Tipps und persönliche Hinweise eines Trainers.

Das erfindungsgemäße System ist geeignet insbesondere auch für mechanische Geräte ohne elektronische Datenspeicherung, da durch den QR-Code und die schnelle Übertragung in das System diese Daten digitalisiert werden.

## Patentansprüche

1. System zur Durchführung von Übungen mit wählbaren Gewichten umfassend:
Einen scannbaren Code, der einem Fitnessgerät zugeordnet ist;
einen Scanner, der durch einen Benutzer zum Scannen vom Code betätigt wird;
ein Mittel, das durch das Betätigen des Scanners den Benutzer auf eine Website automatisch leitet;
eine Datenbank, die mindestens eine Übung, die dem Code zugeordnet ist,
enthält und;
Anzeigen der Übung, die dem Code zugeordnet ist; **gekennzeichnet durch** Mittel zur Eingabe des Gewichts und der Wiederholungszahlen; und
ein Mittel zur Speicherung von Gewicht und von Wiederholungszahlen für die Übung, die dem Code zugeordnet ist.

2. System nach Anspruch 1, **dadurch gekennzeichnet, dass** der Code ein QR-Code ist.

3. System nach Anspruch 2, **dadurch gekennzeichnet, dass** der Scanner in einem Smartphone integriert ist.

4. System nach einem der vorhergehenden Ansprüche, **dadurch gekennzeichnet, dass** die Datenbank persönliche Daten des Benutzers aufweist.

5. System nach einem der vorhergehenden Ansprüche, **dadurch gekennzeichnet, dass** die Datenbank mindestens ein Bild der Übung, die dem Code zugeordnet ist, enthält.

6. System nach einem der vorhergehenden Ansprüche, **dadurch gekennzeichnet, dass** die Datenbank mindestens zwei Bilder der Übung, die dem Code zugeordnet ist, enthält.

7. System nach einem der vorhergehenden Ansprüche, **dadurch gekennzeichnet, dass** die Datenbank mindestens ein Video wie die Übung, die dem Code zugeordnet ist enthält.

8. Aufkleber mit einem QR-Code, der eine URL Adresse kodiert, so dass nach dem Scannen des QR-Code der Benutzer auf eine Website automatisch geleitet word, die mit einer Datenbank verknüpft ist, die mindestens eine Übung, die dem QR-Code zugeordnet ist, enthält und auf der Website diese mindestens eine Übung angezeigt wird, und dass die Website außerdem die Eingabe des Gewichts und der Wiederholungszahlen ermöglicht.
